# EUROPÄISCHE PATENTSCHRIFT

(11) **EP 1 393 693 B1**
(45) Veröffentlichungstag und Bekanntmachung des Hinweises auf die Patenterteilung: **08.03.2006**
(21) Anmeldenummer: 03019246.2
(22) Anmeldetag: 25.08.2003
(51) Int. Cl.: A61D 1/02, A61M 5/46

(54) **Instrument zur Abgabe eines Arzneimittels**
Drug dispensing instrument
Instrument pour distribuer un médicament

(30) Priorität: 28.08.2002 DE 10240201
(43) Veröffentlichungstag der Anmeldung: 03.03.2004
(73) Patentinhaber: ELM-Plastic GmbH, D-54647 Dudeldorf/Eifel (DE)
(72) Erfinder: Lonien, Birgit, 54647 Dudeldorf (DE); Möhs, Sacha, 54647 Dudeldorf (DE)
(74) Vertreter: Serwe, Karl-Heinz

(56) Entgegenhaltungen:
- EP-A- 0 364 817
- EP-A- 0 540 493
- EP-A- 0 904 790
- WO-A-95/01198
- DE-C- 847 473

## Beschreibung

Die Erfindung betrifft ein Instrument zur Abgabe eines für die ärztliche Behandlung eines Euters vorgesehenen Arzneimittels bestehend aus einem Behälter für das Arzneimittel, einer am Behälter angeordneten Kanüle zum Einführen in den Zitzenkanal des Euters und einer am Behälter lösbar angeordneten, die Kanüle umhüllenden Verschlusskappe aus einer oberen Kappe und einer unteren Hülse, wobei die untere Hülse kürzer als die Kanüle und wobei die Verschlusskappe einstückig ausgebildet ist und derart mit einer umlaufenden Sollbruchstelle versehen ist, dass die obere Kappe durch Drehen von der unteren Hülse lösbar ist.

Aus der DE 847 473 ist ein Instrument zur Abgabe eines Arzneimittels bekannt, das aus einem Behälter für das Arzneimittel, einer am Behälter angeordneten Kanüle und einer am Behälter lösbar angeordneten, die Kanüle umhüllenden Verschlusskappe aus einer oberen Kappe und einer unteren Hülse besteht, wobei die untere Hülse kürzer als die Kanüle ist und wobei die Hülse an dem Behälter abschraubbar angeordnet ist. Die obere Kappe und die untere Hülse der Verschlusskappe sind durch eine Sollbruchstelle miteinander verbunden.

Aus der EP 540 493 A1 ist ein Instrument zur Abgabe eines für die ärztliche Behandlung eines Euters vorgesehenen Arzneimittels bekannt, das aus einem Behälter für das Arzneimittel und einer am Behälter angeordneten Kanüle besteht, die von einer Verschlusskappe umhüllt ist, wobei die Verschlusskappe aus oberer Kappe und unterer Hülse besteht, die durch eine Sollbruchstelle miteinander verbunden sind, damit die obere Kappe abtrennbar ist.

Bei der Anwendung des Instrumentes wird je nach der gewünschten Kanülenlänge entweder die gesamte Verschlusskappe aus oberer Kappe und unterer Hülse entfernt, oder nur die obere Verschlusskappe entfernt, so dass zwei vorgegebene Kanülenlängen zur Verfügung stehen, eine kürzere nach Entfernen der oberen Kappe und eine längere nach Entfernen der unteren Hülse mit der oberen Kappe.

Damit die kürzere Kanülenlänge besser an den Zitzenkanal anpassbar ist, wird vorgeschlagen, dass die Hülse an dem Behälter abschraubbar angeordnet ist, so dass die wirksame Länge der Kanüle durch entsprechende Drehung der unteren Hülse gewählt wird.

Nach Entfernen der oberen Kappe kann somit durch entsprechende Drehung der unteren Hülse in Abschraubrichtung die wirksame Länge der Kanüle verkürzt werden. Wird die untere Hülse vollständig abgeschraubt, so steht die gesamte Länge der Kanüle zur Verfügung.

Bei den bisher bekannten Instrumenten wird die obere Kappe bzw. die untere Hülse durch Abbrechen oder Abknicken vom Behälter entfernt. Dies hat jedoch den Nachteil, dass bei diesem Abbrechen oder Abknicken auch die Kanüle verbogen wird, sodass die Kanüle vor der Verwendung des Instrumentes nachgerichtet werden muss. Da dazu die Kanüle berührt werden muss, besteht die Gefahr, dass die Sterilität des Instrumentes verloren geht.

Beim Abdrehen der unteren Hülse oder der oberen Kappe ist jedoch keine Verbiegung der Kanüle zu befürchten, sodass das Instrument steril bleibt.

Vorteilhaft ist am Behälter ein Fortsatz mit einem Außengewinde angeordnet, auf das die ein entsprechendes Innengewinde aufweisende Hülse aufschraubbar ist.

Bei einer weiteren vorteilhaften Ausführungsform ist am Behälter ein Fortsatz mit Außengewinde angeordnet, auf das eine Hülse aufschiebbar ist, die an ihrer Innenseite in Hülsenlängsrichtung verlaufende Nuten hat.

Bei einer bevorzugten Ausführungsform ist die Verschlusskappe einstückig ausgebildet und derart mit einer umlaufenden Sollbruchstelle versehen, dass die obere Kappe durch Drehen von der unteren Hülse lösbar ist.

Vorteilhaft ist die Außenseite der oberen Kappe im Querschnitt mehreckig. Bei einer anderen vorteilhaften Ausführungsform hat die obere Kappe radial verlaufende Flügel:

Vorzugsweise hat die untere Hülse eine umlaufende Grifffläche.

Die Erfindung ist in den Zeichnungen beispielhaft dargestellt. Es zeigen:
- Fig. 1: ein Instrument mit aufgesetzter Verschlusskappe aus oberer Kappe und unterer Hülse,
- Fig. 2: das Instrument nach Fig. 1 bei entfernter oberer Kappe,
- Fig. 3: das Instrument nach Fig. 2 mit teilweise abgeschraubter unterer Hülse und somit verkürzter Kanüle,
- Fig. 4: das Instrument nach Fig. 3 mit vollständig abgeschraubter unterer Hülse und
- Fig. 5 und 6: ein Instrument mit Schraub/Nutverbindung zwischen Behälter und Hülse.

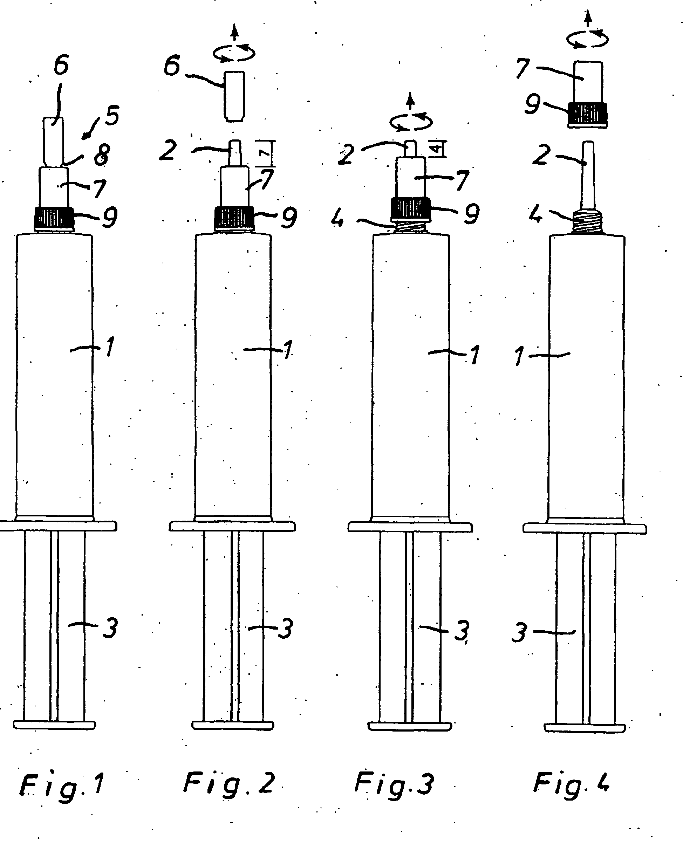

Nach den Figuren 1 bis 4 hat ein Instrument einen Behälter 1 zur Aufnahme eines Arzneimittels. Der Behälter 1 hat an seiner einen Seite eine Kanüle 2 (Figuren 2 bis 4) zur Abgabe des Arzneimittels, das mit Hilfe eines in den Behälter 1 einschiebbaren Druckkolbens 3 aus dem Behälter durch die Kanüle 2 ausgebracht werden kann.

Wie insbesondere die Fig. 4 zeigt, ist die Kanüle 2 von einem am Behälter 1 angeordneten Fortsatz 4 mit einem Außengewinde umgeben, auf die eine Verschlusskappe 5 mit einem entsprechenden Innengewinde aufschraubbar ist, wie dies die Fig. 1 zeigt.

Die Verschlusskappe 5 besteht in bekannter Weise aus einer oberen Kappe 6 und einer unteren Hülse 7, die eine Sollbruchstelle 8 (Fig. 1) aufweist, um die obere Kappe 6 von der unteren Hülse 7 zu trennen. Die umlaufende Sollbruchstelle 8 ist derart ausgebildet, dass die obere Kappe 6 durch Drehen von der unteren Hülse 7 lösbar ist. Dazu ist die Außenseite der oberen Kappe 6 im Querschnitt mehreckig ausgebildet oder hat radial verlaufende Flügel, sodass die Kappe leicht erfaßt und gedreht werden kann.

Durch Drehen der oberen Kappe 6 kann die Kappe von der unteren Hülse 7 gelöst werden und abgezogen werden, sodass die Kanüle 2 teilweise freigegeben wird, wie dies die Fig. 2 zeigt.

Die wirksame Länge der Kanüle 2 kann durch entsprechendes teilweises Abschrauben der unteren Hülse 7 vom Fortsatz 4 beispielsweise von ursprünglich 7 mm auf 4 mm verringert werden, wie dies die Fig. 3 zeigt. Die untere Hülse 7 weist dazu eine umlaufende Grifffläche 9 auf.

Durch vollständiges Abschrauben der unteren Hülse 7 wird die gesamte Länge der Kanüle 2 freigegeben, wie dies die Fig. 4 zeigt.

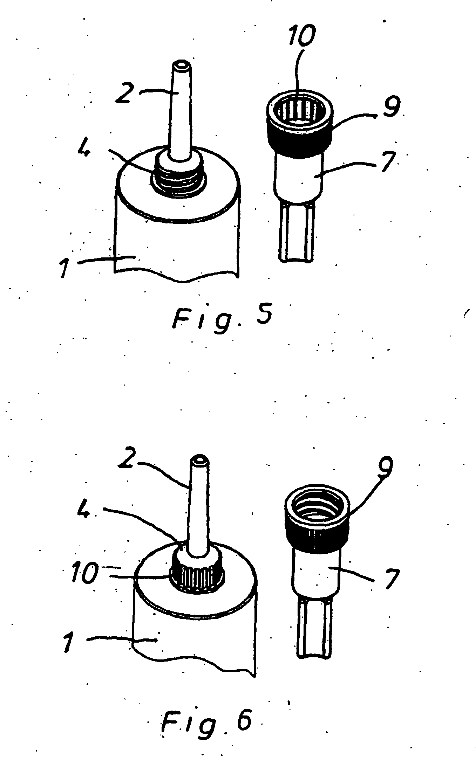

Bei der Ausführungsform eines Instrumentes nach Fig. 5 hat die Hülse 7 an ihrer Innenseite in Hülsenlängsrichtung verlaufende Nuten 10. Bei der Herstellung wird die Hülse 7 dann einfach auf den Fortsatz 4 mit Außengewinde aufgeschoben.

Beim Abdrehen der Hülse 7 schneidet das Außengewinde des Fortsatzes 4 eine Art Gewinde in die Stege zwischen den Nuten 10, sodass eine gezielte Verkürzung der wirksamen Kanülenlänge 2 möglich ist. Es ist auch möglich, die Nuten 10 am Fortsatz 4 anzuordnen und in der Hülse 7 ein Innengewinde anzuordnen, wie dies die Fig. 6 zeigt.

## Patentansprüche

1. Instrument zur Abgabe eines für die ärztliche Behandlung eines Euters vorgesehenen Arzneimittels bestehend aus einem Behälter (1) für das Arzneimittel, einer am Behälter angeordneten Kanüle (2) zum Einführen in den Zitzenkanal des Euters und einer am Behälter (1) lösbar angeordneten, die Kanüle (2) umhüllenden Verschlusskappe (5) aus einer oberen Kappe (6) und einer unteren Hülse (7), wobei die untere Hülse (7) kürzer als die Kanüle (2) und wobei die Verschlusskappe (5), einstückig ausgebildet ist und derart mit einer umlaufenden Sollbruchstelle (8) versehen ist, dass die obere Kappe (6) durch Drehen von der unteren Hülse (7) lösbar ist, **dadurch gekennzeichnet, dass** die Hülse (7) an dem Behälter abschraubbar angeordnet ist, so dass die wirksame Länge der Kanüle durch entsprechende Drehung der unteren Hülse (7) gewählt wird.

2. Instrument nach Anspruch 1, **dadurch gekennzeichnet, dass** am Behälter (1) ein Fortsatz (4) mit einem Außengewinde angeordnet ist, auf das die ein entsprechendes Innengewinde aufweisende Hülse (7) aufschraubbar ist.

3. Instrument nach Anspruch 1, **dadurch gekennzeichnet, dass** am Behälter (1) ein Fortsatz (4) mit Außengewinde angeordnet ist, auf das eine Hülse (7) aufschiebbar ist, die an ihrer Innenseite in Hülsenlängsrichtung verlaufende Nuten (10) hat.

4. Instrument nach einem der Ansprüche 1 bis 3, **dadurch gekennzeichnet, dass** die Verschlusskappe (5) einstückig ausgebildet ist und derart mit einer umlaufenden Sollbruchstelle (8) versehen ist, dass die obere Kappe (6) durch Drehen von der unteren Hülse (7) lösbar ist.

5. Instrument nach Anspruch 4, **dadurch gekennzeichnet, dass** die Außenseite der oberen Kappe (6) im Querschnitt mehreckig ist.

6. Instrument nach Anspruch 4, **dadurch gekennzeichnet, dass** die obere Kappe (6) radial verlaufende Flügel hat.

7. Instrument nach einem der Ansprüche 1 bis 6, **dadurch gekennzeichnet, dass** die untere Hülse (7) eine umlaufende. Grifffläche (9) hat.

## Revendications

1. Instrument permettant de délivrer un médicament destiné au traitement médical d'un pis, ledit instrument étant constitué d'un récipient (1) destiné au médicament, d'une canule (2) placée sur le récipient et destinée à être introduite dans le canal du trayon du pis et d'un capuchon de fermeture (5) qui est placé de façon amovible sur le récipient (1), qui entoure la canule (2) et qui est constitué d'un capuchon supérieur (6) et d'un manchon inférieur (7), le manchon inférieur (7) étant plus court que la canule (2) et le capuchon de fermeture (5) étant conformé d'une seule pièce et doté d'un endroit périphérique de moindre résistance mécanique (8) de sorte que le capuchon supérieur (6) peut être retiré du manchon inférieur (7) par rotation, **caractérisé en ce que** le manchon (7) est placé de façon dévissable sur le récipient de sorte que la longueur efficace de la canule est choisie en tournant de façon correspondante le manchon inférieur (7).

2. Instrument selon la revendication 1, **caractérisé en ce qu'**une saillie (4) dotée d'un filetage extérieur est disposée sur le récipient (1), le manchon (7) comportant un filetage intérieur correspondant et pouvant être vissé sur le filetage extérieur.

3. Instrument selon la revendication 1, **caractérisé en ce qu'**une saillie (4) dotée d'un filetage extérieur est placée sur le récipient (1) et est apte à recevoir à coulissement un manchon (7) qui comporte sur son côté intérieur des gorges (10) s'étendant dans la direction longitudinale du manchon.

4. Instrument selon l'une des revendications 1 à 3, **caractérisé en ce que** le capuchon de fermeture (5) est conformé d'une seule pièce et est doté d'un endroit périphérique de moindre résistance mécanique (8) de sorte que le capuchon supérieur (6) peut être retiré, par rotation, du manchon inférieur (7).

5. Instrument selon la revendication 4, **caractérisé en ce que** le côté extérieur du capuchon supérieur (6) a une section polygonale.

6. Instrument selon la revendication 4, **caractérisé en ce que** le capuchon supérieur (6) comporte des ailettes s'étendant radialement.

7. Instrument selon l'une des revendications 1 à 6, **caractérisé en ce que** le manchon inférieur (7) présente une surface de préhension périphérique (9).

## Claims

1. Instrument for dispensing a drug intended for the medical treatment of an udder consisting of a container (1) for the drug, a cannula (2) for insertion into the teat canal of the udder, which is arranged on the container and a sealing cap (5) surrounding the cannula (2), which is detachably arranged on the container (1) and consists of an upper cap (6) and a lower collar (7), in which the lower collar (7) is shorter than the cannula (2) and in which the sealing cap (5) is made in one piece and provided with a circumferential predetermined breaking point (8) in such a way that the upper cap (6) can be detached from the lower collar (7) by means of rotation, **characterised in that** the collar (7) is arranged on the container so that it can be unscrewed, so that the effective length of the cannula is selected by the lower collar (7) being rotated in the appropriate way.

2. Instrument according to claim 1 **characterised in that** an appendage (4) with an external screw thread is arranged on the container (1), on which a collar (7) that has a corresponding internal screw thread can be screwed.

3. Instrument according to claim 1 **characterised in that that** an appendage (4) with an external screw thread is arranged on the container (1), onto which a collar (7), which has grooves (10) on the inside running in the longitudinal direction of the collar, can be slid.

4. Instrument according to one of claims 1 to 3 **characterised in that** the sealing cap (5) is made in one piece and is provided with a circumferential predetermined breaking point (8) in such a way that the upper cap (6) can be detached from the lower collar (7) by means of rotation.

5. Instrument according to claim 4 **characterised in that** the outside of the upper cap is (6) is polygonal in cross section.

6. Instrument according to claim 4 **characterised in that** the upper cap (6) has vanes that run radially.

7. Instrument according to one of claims 1 to 6 **characterised in that** the lower collar (7) has a circumferential gripping surface (9).
